# EUROPEAN PATENT SPECIFICATION

(11) **EP 1 131 130 B1**
(45) Date of publication and mention of the grant of the patent: **18.08.2004**
(21) Application number: 00954847.0
(22) Date of filing: 08.09.2000
(51) Int. Cl.: A61N 1/16

(54) **DEVICE FOR PROTECTIVE CONDITIONING OF ELECTROMAGNETIC FIELDS PRODUCED BY NON-IONOGENIC RADIATIONS SUCH AS RADIOFREQUENCY EMISSIONS OR THE LIKE**
GERÄT ZUR SCHÜTZENDEN BEEINFLUSSUNG ELEKTROMAGNETISCHER FELDER, WIE SIE DURCH NICHT-IONISIERENDE STRAHLUNG WIE Z.B. RADIOFREQUENTE EMISSIONEN O.Ä. HERVORGERUFEN WERDEN
DISPOSITIF DE PROTECTION DESTINE AU TRAITEMENT DES CHAMPS ELECTROMAGNETIQUES PRODUITS PAR LES RAYONNEMENTS NON IONISANTS, TELS QUE LES EMISSIONS DU TYPE RADIOFREQUENCES OU ANALOGUES

(30) Priority: 10.09.1999 IT PD990202
(43) Date of publication of application: 12.09.2001
(73) Proprietor: Bonomo, Dario, 35042 Este (Padova) (IT)
(72) Inventor: Bonomo, Dario, 35042 Este (Padova) (IT)
(74) Representative: Modiano, Guido, Dr.-Ing.
(86) International application number: PCT/IB2000/001272
(87) International publication number: WO 2001/019451

(56) References cited:
- EP-A- 0 280 644
- WO-A-95/33515
- DE-U- 29 612 876

## Description

### TECHNICAL FIELD

The present invention relates to a device for human-protective conditioning of electromagnetic fields produced by non-ionogenic radiations such as radiofrequency emissions or the like.

### BACKGROUND ART

It is known that a radiofrequency source produces, in the surrounding atmosphere, a magnetic field which is characterized by a specific periodic sinusoidal shape and by a specific frequency, said frequency being understood as a complete cycle from the initial zero point, i.e., where the voltage is zero, to the next zero point, passing from a maximum peak of positive value to a maximum peak of negative value.

The generated magnetic field is measured with appropriate instruments which measure its potential in volts/square meter of its incidence or in the equivalent of the AC voltage collected by a hypothetical receiving element with an area of 1 square meter.

Such voltage, which is present in the surrounding atmosphere, at values above thresholds set by competent institutional authorities (a typical value is 6 volts/square meter for inhabited areas) must unquestionably be considered harmful for the health of living beings which are directly exposed to it.

In particular, the harmfulness of high-frequency magnetic fields, i.e., alternating ones, is indeed located in organic cells whose life depends, as is known, on electrical parameters which are altered by the presence of the above-mentioned magnetic fields.

In particular, anomalies at the nervous level have been noted which, as immersion in magnetic fields persists, can cause, in the most severe cases, irreversible imbalances in the cell balance, ultimately leading to disorders even of carcinogenic origin.

However, it is currently substantially impossible to eliminate devices which in some way generate radiofrequencies and therefore produce the consequent electromagnetic fields.

The number and usefulness of devices having these characteristics are in fact presently such as to direct research toward forms of passive protection rather than toward eliminating said devices.

WO-A-9 533 515 discloses a device for the protective conditioning of electromagnetic fields produced by non-ionogenic radiations such as radiofrequency emissions or the like, which device comprises, inside an annular element comprising a conductive powder, a permanent magnet and a conducting element shaped like a closed spiral.

### DISCLOSURE OF THE INVENTION

The aim of the present invention is to provide a device for human-protective conditioning of electromagnetic fields produced by non-ionogenic radiations, such as radiofrequency emissions or the like, which indeed ensures reduction of the electromagnetic field that is present within a predefined radius of influence so as to generate a space which is free of electromagnetic pollution for the user.

Within the scope of this aim, an important object of the present invention is to provide a device which is highly effective but also highly portable so that it can be advantageously applied, without compromising functionality, even to small devices such as cellular telephones or the like.

Another object of the present invention is to provide a device which is entirely devoid of negative side effects for the user.

Another object of the present invention is to provide a device whose effectiveness persists for a long time at costs which are substantially competitive with respect to more complex reduction methods.

Another object of the present invention is to provide a device which can be manufactured with known technologies and equipment.

This aim, these objects and others which will become better apparent hereinafter are achieved by a device for the protective conditioning of electromagnetic fields produced by non-ionogenic radiations such as radiofrequency emissions or the like, which device comprises, inside an annular element made of conducting material and at least partially gold-plated, a permanent magnet and a ceramic support with which a conducting element shaped like a closed spiral is associated.

### BRIEF DESCRIPTION OF THE DRAWINGS

Further characteristics and advantages of the present invention will become better apparent from the description of an embodiment thereof, illustrated only by way of non-limitative example in the accompanying drawings, wherein:
Figure 1 is an axonometric view of a device according to the invention;
Figure 2 is an orthographic projection view of part of the device of Figure 1;
Figure 3 is another orthographic projection view of the device of Figure 1;
Figure 4 is a sectional view of the device of Figure 1;
Figure 5 is a view of a device, identical to the one shown in Figure 1, applied to a cellular telephone.

### WAYS OF CARRYING OUT THE INVENTION

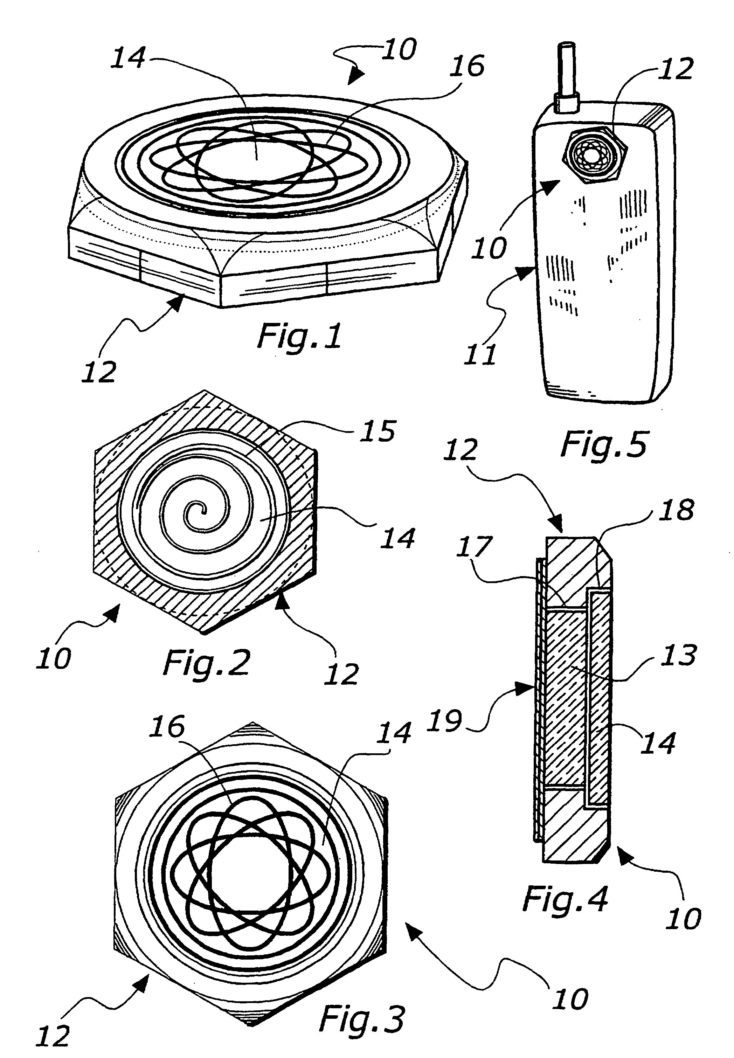

With particular reference to Figures 1 to 5, a device for human-protective conditioning of electromagnetic fields produced by non-ionogenic radiations such as radiofrequency emissions or the like, according to the invention, is generally designated by the reference numeral 10.

The device 10, which in particular in Figure 5 is shown applied to a cellular telephone generally designated by the reference numeral 11, comprises, inside a gold-plated annular element 12 made of conducting material (in this case copper), a permanent magnet 13 which provides a polarity inverter and a ceramic support 14 with which a conducting element 15 (gold leaf) shaped like a closed spiral is associated on the side directed toward said magnet 13.

The magnet 13 must be arranged so that the positive pole lies toward the conducting element 15.

The spiral of the element 15, in this configuration of the device, should conveniently be left-handed.

If the element 15 is arranged, on the support 14, on the opposite side with respect to the magnet 13, the spiral should conveniently be right-handed.

In this embodiment, another conducting element 16 is associated on the ceramic support 14, which is disk-shaped, at the face that lies opposite the one with which the first conducting element 15 is associated; the conducting element 16 is shaped so as to form a plurality of mutually intersecting ellipses.

The element 12, in this embodiment, is shaped externally so as to form a hexagonal perimeter, while on the inside the surfaces and the definitions of the hole are shaped so as to form two seats having different diameters, designated by the reference numerals 17 and 18 respectively, which are adapted to accommodate the above-cited magnet 13 and ceramic support 14.

In particular, the ceramic support 14 is arranged so that the conducting element 16 is directed outward.

The device 10 also comprises means for anchoring to the frame or to supports of devices that produce electromagnetic emissions; in this case, such means are constituted by a double-adhesive element 19.

In practice, the operation of the device 10 is as follows: there is a known fundamental principle in the electrotechnical field according to which any coil of conducting material, immersed in an alternating magnetic field, becomes the seat of an induced current which is proportional to said magnetic field and becomes the source of an alternating magnetic field which is proportional but inverse with respect to the current induced by the primary magnetic field.

This physical phenomenon is commonly known and utilized by every rotary and static electrical machine.

In this sense, therefore, the assembly constituted by the magnet 13, the first conducting element 15 and the shell 12 forms a source of a magnetic field which is induced by the one generated by the polluting device but is opposite in value, so as to have, as a result, an attenuation or elimination of interference.

The above-mentioned result can be demonstrated analytically by knowing the forces involved and applying one of the vector systems commonly used in the electrotechnical or radiotechnical field.

More specifically, the annular shell 12 constitutes the main coil that collects the polluting high-frequency magnetic field, while the permanent magnet 13 represents the unit of fixed contrasting force which is enhanced, increasing or reducing its value in gauss, cyclically by the force generated by the coil.

Moreover, the gold plating is meant to constitute an element of maximum conductivity in order to provide optimum dissipation of the higher surface current density; in this regard, in higher-value devices the annular element 12 can be made entirely of gold.

The above is also linked to the presence of alternating high frequencies, which trigger a skin effect, and the electrical charges tend to be arranged radially with respect to the cross-section of the conductor, hindering the flowing current and thus producing a virtual resistivity which is counterproductive due to its thermal effects.

The first conducting element 15 defines the secondary spiral of gold leaf on ceramic, which is meant to enhance the energy exchange of the contrasting field with the atmosphere and constitutes, in practice, a true transmission antenna.

The second conducting element 16 further ensures a further increase in the reduction potential of the above-described elements.

As a whole, therefore, a high-contrast electromagnetic field is generated by systematically converting the initial wave into another identical wave which is identical but entirely opposite in value.

On the environmental level, this is perceived as a true "cleaning", with radial and drastic reductions in the electromagnetic pollution produced by appliances that use electrical devices and radiofrequency.

In practice, it has been observed that the present invention has achieved the intended aim and objects.

In particular, it should be noted, in relation to the above, that the device according to the invention ensures, without any kind of negative side effect for the user, a high reduction if not, in the best cases, complete elimination of polluting electromagnetic fields generated by radiofrequency emissions.

In this regard, therefore, the device according to the invention ensures, from the electromagnetic point of view, a high environmental healthfulness which has a deep and positive effect on the cell balance of the living beings that are present in such environment.

It should also be noted that all this is achieved by a device which is very compact and can be easily applied practically to any type of appliance, even small ones such as a cellular telephone.

It should also be noted that the device according to the invention can be manufactured flexibly even in models with different commercial values and power ratings so as to ensure usability by any kind of user.

## Claims

1. A device for the protective conditioning of electromagnetic fields produced by non-ionogenic radiations such as radiofrequency emissions or the like, which device comprises, inside an annular element (12) made of conducting material and at least partially gold-plated, a permanent magnet (13) and a ceramic support (14) with which a conducting element shaped like a closed spiral (15) is associated.

2. The device according to claim 1, **characterized in that** said magnet is arranged so that the positive pole is directed toward said conducting element.

3. The device according to claim 1, **characterized in that** said spiral conducting element is arranged toward said magnet.

4. The device according to claims 1 and 3, **characterized in that** said spiral of said conducting element is left-handed.

5. The device according to claim 1, **characterized in that** if said conducting element is arranged on said ceramic support on the opposite side with respect to said magnet said spiral is right-handed.

6. The device according to claim 1, **characterized in that** said ceramic support is substantially circular and **in that** a second conducting element (16), shaped so as to form mutually intersecting shapes, is applied on a face which lies opposite the one to which said conducting element is applied.

7. The device according to claim 6, **characterized in that** said second conducting element is shaped so as to form a plurality of mutually intersecting ellipses.

8. The device according to one or more of the preceding claims, **characterized in that** said permanent magnet is disk-shaped.

9. The device according to one or more of the preceding claims, **characterized in that** said annular shell is shaped internally so as to form two connected seats (17, 18) having different diameters which are adapted to respectively accommodate said permanent magnet and said ceramic support.

10. The device according to claim 9 as far as it depends from claim 6 or 7, **characterized in that** said ceramic support is accommodated in said seat so as to expose externally said second conducting element.

11. The device according to one or more of the preceding claims, **characterized in that** it comprises means for anchoring to a frame or to supports of devices that emit electromagnetic fields or radiofrequencies.

12. The device according to claim 11, **characterized in that** said anchoring means are constituted by a double-adhesive element (19) associated with said annular element.

13. The device according to one or more of the preceding claims, **characterized in that** said annular element is made of solid gold.

## Patentansprüche

1. Vorrichtung zur schützenden Konditionierung von elektromagnetischen Feldern, die durch nicht-ionenerzeugende Strahlungen erzeugt werden, wie Radiofrequenz-Emissionen oder dergleichen, welche Vorrichtung im Inneren eines aus leitendem Material hergestellten und mindestens teilweise vergoldeten ringförmigen Elements (12) einen Dauermagneten (13) und einen Keramikträger (14) umfasst, mit dem ein wie eine geschlossene Spirale (15) geformtes leitendes Element verbunden ist.

2. Vorrichtung nach Anspruch 1, **dadurch gekennzeichnet, dass** der Magnet so angeordnet ist, dass der positive Pol zu dem leitenden Element hin gerichtet ist.

3. Vorrichtung nach Anspruch 1, **dadurch gekennzeichnet, dass** das spiralige leitende Element zu dem Magneten hin angeordnet ist.

4. Vorrichtung nach den Ansprüchen 1 und 3, **dadurch gekennzeichnet, dass** die Spirale des leitenden Elements linkshändig ist.

5. Vorrichtung nach Anspruch 1, **dadurch gekennzeichnet, dass** wenn das leitende Element auf der in Bezug zu dem Magneten entgegengesetzten Seite auf dem Keramikträger angeordnet ist, die Spirale rechtshändig ist.

6. Vorrichtung nach Anspruch 1, **dadurch gekennzeichnet, dass** der Keramikträger im Wesentlichen kreisförmig ist, und dass ein zweites leitendes Element (16), das so geformt ist, dass sich gegenseitig überlagernde Formen gebildet werden, auf einer Seite angebracht ist, die zu der einen, auf der das leitende Element angebracht ist, entgegengesetzt ist.

7. Vorrichtung nach Anspruch 6, **dadurch gekennzeichnet, dass** das zweite leitende Element so geformt ist, dass es eine Mehrzahl von sich gegenseitig durchdringenden Ellipsen bildet.

8. Vorrichtung nach einem oder mehreren der vorangehenden Ansprüche, **dadurch gekennzeichnet, dass** der Dauermagnet scheibenförmig ist.

9. Vorrichtung nach einem oder mehreren der vorangehenden Ansprüche, **dadurch gekennzeichnet, dass** der ringförmige Mantel im Inneren so geformt ist, dass er zwei miteinander verbundene Sitze (17, 18) mit unterschiedlichen Durchmessern bildet, die angepasst sind, um den Dauermagneten bzw. den Keramikträger unterzubringen.

10. Vorrichtung nach Anspruch 9, soweit er auf Anspruch 6 oder 7 rückbezogen ist, **dadurch gekennzeichnet, dass** der Keramikträger so in dem Sitz untergebracht ist, dass das zweite leitende Element nach außen zu frei liegt.

11. Vorrichtung nach einem oder mehreren der vorangehenden Ansprüche, **dadurch gekennzeichnet, dass** sie Einrichtungen zur Verankerung an einem Gestell oder an Halterungen von Vorrichtungen umfasst, die elektromagnetische Felder oder Radiofrequenzen emittieren.

12. Vorrichtung nach Anspruch 11, **dadurch gekennzeichnet, dass** die Verankerungseinrichtungen von einem doppeltklebenden Element (19) gebildet werden, das mit dem ringförmigen Element verbunden ist.

13. Vorrichtung nach einem oder mehreren der vorangehenden Ansprüche, **dadurch gekennzeichnet, dass** das ringförmige Element aus massivem Gold hergestellt ist.

## Revendications

1. Dispositif pour le conditionnement protecteur de champs électromagnétiques produits par des rayonnements non ionisants tels que des émissions de radiofréquence ou équivalent, lequel dispositif comprend, à l'intérieur d'un élément annulaire (12) composé d'un matériau conducteur et au moins partiellement doré, un aimant permanent (13) et un support en céramique (14) auquel un élément conducteur façonné comme une spirale fermée (15) est associé.

2. Dispositif selon la revendication 1, **caractérisé en ce que** ledit aimant est agencé de sorte que le pôle positif est dirigé vers ledit élément conducteur.

3. Dispositif selon la revendication 1, **caractérisé en ce que** ledit élément conducteur en spirale est agencé vers ledit aimant.

4. Dispositif selon les revendications 1 et 3, **caractérisé en ce que** ladite spirale dudit élément conducteur est en rotation à gauche.

5. Dispositif selon la revendication 1, **caractérisé en ce que** si ledit élément conducteur est agencé sur ledit support céramique sur le côté opposé par rapport audit aimant ladite spirale est en rotation à droite.

6. Dispositif selon la revendication 1, **caractérisé en ce que** ledit support en céramique est sensiblement circulaire et **en ce qu'**un deuxième élément conducteur (16), façonné de manière à former des formes se croisant mutuellement, est appliqué sur une face qui se trouve à l'opposé de celle sur laquelle ledit élément conducteur est appliqué.

7. Dispositif selon la revendication 6, **caractérisé en ce que** ledit deuxième élément conducteur est façonné de manière à former une pluralité d'ellipses se croisant mutuellement.

8. Dispositif selon l'une ou plusieurs des revendications précédentes, **caractérisé en ce que** ledit aimant permanent est en forme de disque.

9. Dispositif selon l'une ou plusieurs des revendications précédentes, **caractérisé en ce que** ladite coquille annulaire est façonnée à l'intérieur de manière à former deux logements connectés (17, 18) présentant des diamètres différents qui sont adaptés pour recevoir respectivement ledit aimant permanent et ledit support en céramique.

10. Dispositif selon la revendication 9 dans la mesure où elle dépend de la revendication 6 ou 7, **caractérisé en ce que** ledit support en céramique est reçu dans ledit logement de manière à exposer à l'extérieur ledit deuxième élément conducteur.

11. Dispositif selon l'une ou plusieurs des revendications précédentes, **caractérisé en ce qu'**il comprend des moyens pour ancrer à un châssis ou à des supports des dispositifs qui émettent des champs électromagnétiques ou des radiofréquences.

12. Dispositif selon la revendication 11, **caractérisé en ce que** lesdits moyens d'ancrage sont constitués par un élément adhésif double (19) associé audit élément annulaire.

13. Dispositif selon l'une ou plusieurs des revendications précédentes, **caractérisé en ce que** ledit élément annulaire est composé d'or massif.
